# EUROPÄISCHE PATENTSCHRIFT

(11) **EP 2 222 644 B1**
(45) Veröffentlichungstag und Bekanntmachung des Hinweises auf die Patenterteilung: **03.08.2011**
(21) Anmeldenummer: 08857143.5
(22) Anmeldetag: 21.11.2008
(51) Int. Cl.: C07D 233/32

(54) **VERFAHREN ZUR HERSTELLUNG VON VORLÄUFERN FÜR L-2-[18F]FLUOR-PHENYLALANIN UND 6-[18F]FLUOR- L-META-TYROSIN UND DEREN ALPHA-METHYLIERTEN DERIVATEN, SOWIE DIE VORLÄUFER**
METHOD FOR PRODUCING PRECURSORS FOR L-2-[18F]FLUOROPHENYLALANINE AND 6-[18F]FLUORO-L--META-TYROSINE AND THE ALPHA -METHYLATED DERIVATIVES THEREOF AND THE PRECURSORS
PROCÉDÉ POUR LA PRODUCTION DE PRÉCURSEURS DE L-2-[18F]FLUOROPHÉNYLALANINE ET DE 6-[18F]FLUORO- L-MÉTA-TYROSINE ET DE LEURS DÉRIVÉS ALPHA-MÉTHYLÉS ET LES PRÉCURSEUR

(30) Priorität: 07.12.2007 DE 102007059314
(43) Veröffentlichungstag der Anmeldung: 01.09.2010
(60) Teilanmeldung: 11001786.0
(73) Patentinhaber: Forschungszentrum Jülich GmbH, 52425 Jülich (DE)
(72) Erfinder: WAGNER, Franziska, 85599 Hergolding (DE); ERMERT, Johannes, 51109 Köln (DE); COENEN, Heinrich, Hubert, 41515 Grevenbroich (DE)
(86) Internationale Anmeldenummer: PCT/DE2008/001936
(87) Internationale Veröffentlichungsnummer: WO 2009/071050

(56) Entgegenhaltungen:
- WO-A-2005/037737
- TIERLING T ET AL: "A new nucleophilic asymetric synthesis of 6-[18F]Fluoro-DOPA" JOURNAL OF LABELLED COMPOUNDS AND RADIOPHARMACEUTICALS, JOHN WILEY, CHICHESTER, GB, Bd. 44, Nr. suppl. 1, 1. Januar 2001 (2001-01-01), Seiten 145-146, XP009113825 ISSN: 0362-4803 in der Anmeldung erwähnt
- NAMAVARI M ET AL: "Synthesis of 6-[<18>F] and 4-[<18>F]fluoro-l-m-tyrosines via regioselective radiofluorodestannylation" APPLIED RADIATION AND ISOTOPES, ELSEVIER, OXFORD, GB, Bd. 44, Nr. 3, 1. März 1993 (1993-03-01), Seiten 527-536, XP024685368 ISSN: 0969-8043 [gefunden am 1993-03-01]
- PAGES T ET AL: "Fluorination of aromatic compounds from 1-aryl-3,3-dimethyltriazenes and fluoride anions in acidic medium - 2. Synthesis of (S)-[<18>F]-3-fluoro-alpha-methylphenylala nine" JOURNAL OF FLUORINE CHEMISTRY, ELSEVIER, NL, Bd. 107, Nr. 2, 1. Februar 2001 (2001-02-01), Seiten 329-335, XP004315016 ISSN: 0022-1139
- SHEN ET AL: "Decarbonylation of multi-substituted [<18>F]benzaldehydes for modelling syntheses of <18>F-labelled aromatic amino acids" APPLIED RADIATION AND ISOTOPES, ELSEVIER, OXFORD, GB, Bd. 65, Nr. 11, 17. Oktober 2007 (2007-10-17), Seiten 1227-1231, XP022302102 ISSN: 0969-8043
- PLENEVAUX A ET AL: "Synthesis of non-activated <18>F-fluorinated aromatic compounds through nucleophilic substitution and decarboxylation reactions" APPLIED RADIATION AND ISOTOPES, INTERNATIONAL JOURNAL OFRADIATION APPLICATIONS AND INSTRUMENTATION, PART A, PERGAMON PRESS LTD, GB, Bd. 43, Nr. 8, 1. August 1992 (1992-08-01), Seiten 1035-1040, XP024706845 ISSN: 0883-2889 [gefunden am 1992-08-01]

## Beschreibung

Die Erfindung betrifft ein Verfahren zur Herstellung von Vorläufern für 2-[18F]Fluorphenylalanin (2-[18F]FPhe) und 6-[18F]Fluor-L-meta-tyrosin (6-[18F]FMT) und deren αmethylierten Derivaten sowie den Vorläufern.

Aus dem Jahre 2001 ist eine Veröffentlichung von T.Tierling, K. Hamacher und H. H. Coenen mit dem Titel "A new nucleophilic asymetric synthesis of 6-[18F]Fluoro-DOPA" bekannt, die in J. Label. Compds. Radiopharm. 44, Suppl. 1 erschienen ist und in der ein Verfahren zur Herstellung eines [18F]FDOPA-Vorläufers und dessen Umsetzung zu 6-[18F]Fluoro-DOPA beschreibt. Nach diesem Verfahren wird ein Vorläufer durch nukleophile Substitution mit einem K2CO3 Kryptand-Komplex zu [18F]FDOPA umgesetzt. Das Produkt wird in einer Enantiomerenreinheit von 85% erhalten.

Eine elektrophile Synthese von 6-[¹⁸F]⁻ und 4-[¹⁸F]Fluor- L-meta-tyrosin ist aus dem Artikel Synthesis of 6-[¹⁸F]⁻ und 4-[¹⁸F]Fluoro- L-meta-tyrosines via Regioselective Radiofluorodestannylaton der Autoren M. Namavari et. al. in Appl. Radiat. Isot. Vol 44, -NR. 3, pp 527-536 aus dem Jahre 1993 bekannt.

Die Veröffentlichung "Fluorination of aromatic compounds from 1-aryl-3,3-dimethyltriazenes and fluoride anions in acidic medium 2. Synthesis of (S)- [18F]-3-fluoro-α-methylphenylalanine" von Thierry Pages et. al im Journal of Fluorine Chemistry 107 (2001) S. 329-335 offenbart Vorläufer und Herstellungsverfahren von [¹⁸F] fluormarkierten aromatischen L-Aminosäuren.

Die nach dem Stand der Technik bekannten Verfahren zur Herstellung von 2-[¹⁸F]FPhe und 2-[¹⁸F]FMT-Vorläufern führen zu relativ geringen radiochemischen Ausbeuten und sind daher mit hohem Kostenaufwand verbunden.

Es ist daher die Aufgabe der Erfindung, Vorläufern von 2-[¹⁸F]FPhe, 6-[¹⁸F]FMT und deren α-methylierten Derivaten und ein Verfahren zur Herstellung deren Vorläufer von 2-[¹⁸F]FPhe, 6-[¹⁸F]FMT und deren α-methylierten Derivaten zur Verfügung zu stellen, welches zu größeren radiochemischen Ausbeuten und zu einer größeren Enantiomerenreinheit führt. Außerdem soll das Verfahren für einen automatisierten Synthesebetrieb geeignet sein.

Die Aufgabe wird erfindungsgemäß gelöst mit den Merkmalen der unabhängigen Ansprüche.

Mit dem erfindungsgemäßen Verfahren und den Vorläufern ist es nunmehr möglich, 2-[¹⁸F]FPhe, 6-[¹⁸F]FMT und deren α-methylierte Derivate in nur drei radioaktiven Schritten enantiomerenrein herzustellen. Die Synthese kann automatisiert durchgeführt werden und führt zu Enantiomerenreinheiten von ≥ 98 %.

Die Figuren zeigen Reaktionschemata für die Herstellung der erfindungsgemäßen Vorläufer sowie für die Herstellung der Zielverbindungen 2-[¹⁸F]FPhe und 6-[¹⁸F]FMT und deren αmethylierte Derivate.

Es zeigt:
- Fig.1:: Ein allgemeines Reaktionsschema für die Herstellung des Vorläufers.

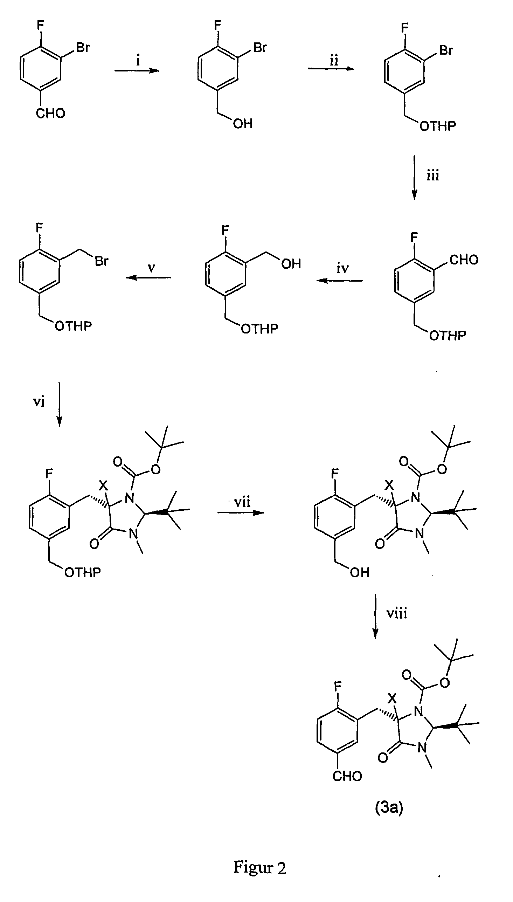

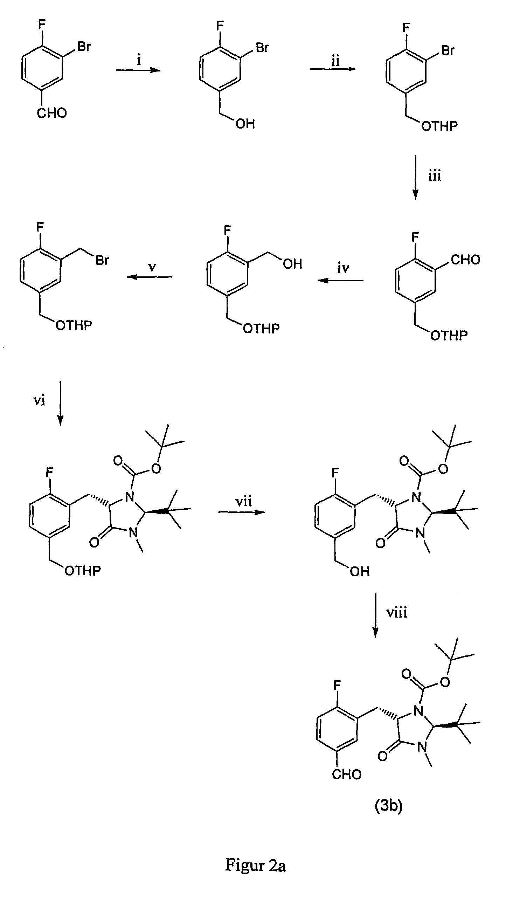

- Fig.2:: Ein Reaktionsschema für die Synthese des Vorläufers, mit einzelnen Reaktionsschritten.
- Fig.2a:: Ein spezielles Reaktionsschema für die Synthese des Vorläufers, mit einzelnen Reaktionsschritten.

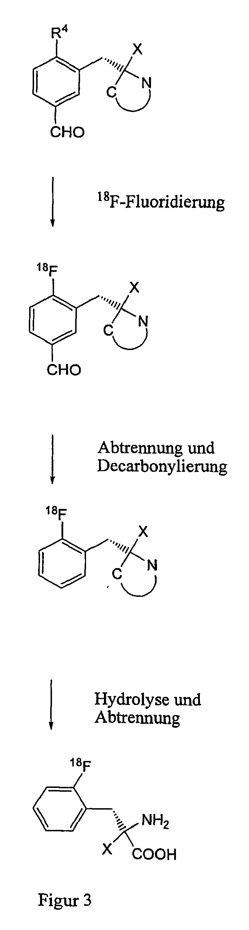

- Fig.3:: Allgemeine Schritte zur Herstellung von 2-[¹⁸F]FPhe und des α- methylierten Derivates.

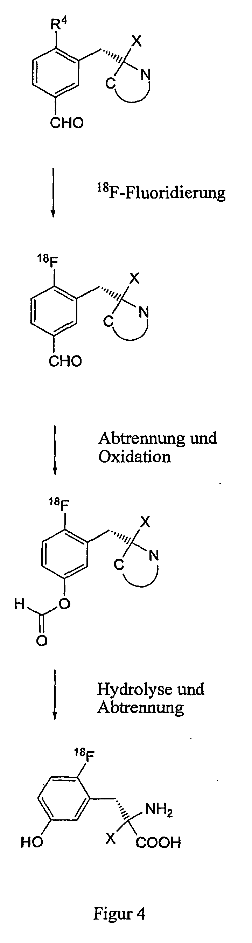

- Fig.4:: Allgemeine Schritte zur Herstellung von 6-[¹⁸F]FMT und des α- methylierten Derivates.

Formel 1 zeigt die Struktur von 2-[¹⁸F]FPhe und des α-methylierten Derivates. In ihr ist X = H oder CH₃.

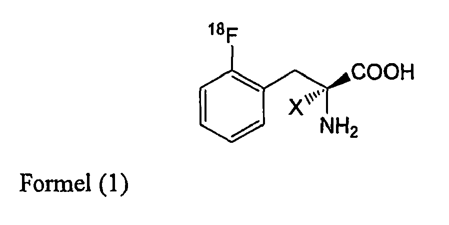

Formel 2 zeigt die Struktur von 6-[¹⁸F]FMT und des α-methylierten Derivates. In ihr ist X = H oder CH₃.

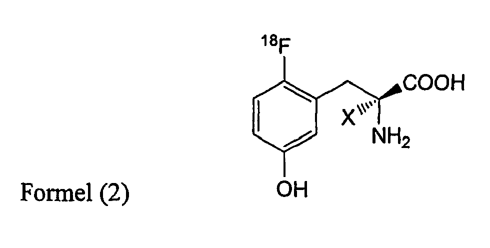

Formel (3) zeigt die Struktur des Vorläufers. In ihr ist X = H oder CH₃.

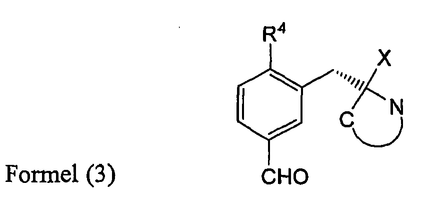

Im Folgenden soll die Erfindung in ihrer allgemeinen Form beschrieben werden:

In den Formeln sowie in den Figuren können als Substituenten R*ⁿ*, X, folgende Gruppen angegeben werden:
- R¹: = Br, I
- R²: = Tetrahydropyranyl (THP), Methylthiomethyl (MTM), Methoxymethyl (MOM), TBDMS, TBDPS, allgemein Silylschutzgruppen
- R³: = (S)-BOC-BMI: (S)-1-(tert.-Butoxycarbonyl)-2-tert.-butyl-3-methyl-4- imi- dazolidinon , (S)-Cbz-BMI: (S)-1-(Benzoylcarbonyl)-2-tert.-butyl-3-methyl-4- midazolidinon, (S)-BDI: (S)-tert.-Butyl 2-tert.-butyl-4-methoxy-2,5-dihydroimidazol-1- carboxylat, Methyl-(S)-BOC-BMI: (2S, 5R)-tert.-Butyl-2-tert.-butyl-3,5-dimethyl-4- oxoimidazolidin-1-carboxylat , Methyl-(S)-Cbz-BMI: (S)-1-(Benzoylcarbonyl)-2-tert.-butyl-3,5-dimethyl-4- imidazolidinon oder Methyl-(S)-BDI: (S)-tert.-Butyl-2-tert.-butyl-5-methyl-4-methoxy-2- hydroimidazol-1-carboxylat Formelschreibweise: 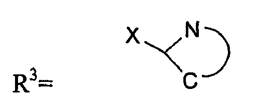
- R⁴: = nukleophile Abgangsgruppe z. B. F, Br, Cl, NO₂ oder -NR₃⁺ mit R = Alkyl, z. B. CH₃, C₂H₅. beispielsweise CH₃
- X: = H oder CH₃

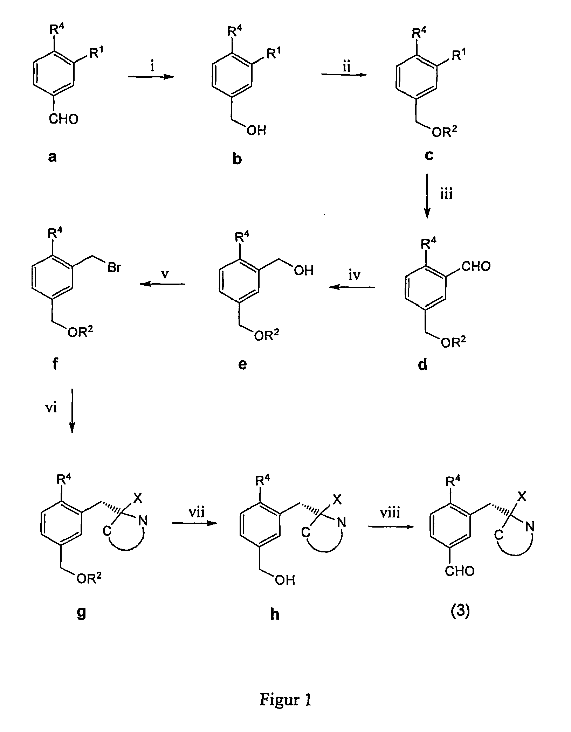

In Figur 1 ist ein allgemeines Reaktionsschema angegeben, nach dem der Vorläufer für 2-[¹⁸F]FPhe und 6-[¹⁸F]FMT gemäß Formel 3 hergestellt werden kann.

Die Aldehylgruppe von Verbindung a wird in Schritt i reduziert.

Als Reduktionsmittel kann beispielsweise ein Metallhydrid, wie Natriumborhydrid oder Lithiumaluminiumhydrid eingesetzt werden.

Geeignete Lösungsmittel sind insbesondere bei der Verwendung von Natriumborhydrid, Methanol oder auch andere Alkohole, beispielsweise Ethanol, Propanol oder Isopropanol.

Vorzugsweise wird die Reaktion bei Raumtemperatur durchgeführt.

Mit diesem Herstellungsverfahren kann die Verbindung b als Edukt für das erfindungsgemäße Herstellungsverfahren des Vorläufers beispielhaft hergestellt werden. Jedoch können für b auch andere Synthesewege eingeschlagen werden.

In den resultierenden Alkohol b wird erfindungsgemäß in Schritt ii eine Schutzgruppe eingeführt.
Als Schutzgruppen kann THP, MTM, TBDMS, TBDPS, allgemein Silylschutzgruppen oder MOM eingesetzt werden.
Hierzu kann p-Toluolsulfonsäure als Katalysator hinzu gegeben werden.

Als Lösungsmittel kann Dichlormethan, DMF oder Tetrahydrofuran eingesetzt werden.

Die Reaktionstemperatur liegt vorzugsweise zwischen 0°C und Raumtemperatur, beispielsweise 17°C bis 25°C.

In einem folgenden Schritt iii wird bei Verbindung c der Substituent R¹ durch eine Formylgruppe ersetzt.

Die Formylierung kann dabei beispielsweise mit Anilid, Formylpiperidin oder Dimethylformamid in Anwesenheit von Metallierungsgreagentien, wie sec.-Butyllithium, n-Buyllithium, tert.-Butyllitium, Lithium oder Magnesium erfolgen.

Als Lösungsmittel kann Tetrahydrofuran oder beispielsweise ein anderer Ether eingesetzt werden.

Die Reaktion kann in einem Temperaturbereich zwischen -20°C und -80°C, vorzugsweise zwischen -50°C und -80°C besonders bevorzugt bei -78°C, also Trockeneistemperatur durchgeführt werden.

Die resultierende Verbindung d wird in einem folgenden Schritt iv zu einem Alkohol e reduziert.

Als Reduktionsmittel kann beispielsweise ein Metallhydrid, wie Natriumborhydrid, Lithiumaluminiumhydrid eingesetzt werden.

Geeignete Lösungsmittel sind insbesondere bei der Verwendung von Natriumborhydrid Methanol oder auch andere Alkohole, wie Ethanol, Propanol oder Isopropanol.

Vorzugsweise wird die Reaktion bei Raumtemperatur durchgeführt.

Der Alkohol e wird in der Folgereaktion v zu Verbindung f halogeniert oder tosyliert, wobei die Tosylgruppe in an die Stelle des Br in Formel f tritt.

Hierzu können vorzugsweise Tetrabrommethan in Gegengwart von Triphenylphosphin als Sauerstofffänger eingesetzt werden.

Als Lösungsmittel können Dichlonnethan oder allgemein halogenierte Kohlenwasserstoffe eingesetzt werden.

Die bevorzugte Temperatur liegt bei 0°C bis ca. 4°C.

Verbindung f wird in Reaktionsschritt vi mit einem chiralen Aminosäurereagenz umgesetzt. Hierzu wird Verbindung f mit (S)-BOC-BMI: (S)-1-(tert.-Butoxycarbonyl)-2-tert.-butyl-3-methyl-4-imidazolidinon, (S)-Cbz-BMI: (S)-1-(Benzoylcarbonyl)-2-tert.-butyl-3-methyl-4-imidazolidinon, (S)-BDI: (S)-tert.-Butyl 2-tert.-butyl-4-methoxy-2,5-dihydroimidazol-1-carboxylat, Methyl-(S)-BOC-BMI: (2S,5R)-tert.-Butyl-2-tert.-butyl-3,5-dimethyl-4-oxoimidazolidin-1-carboxylat, Methyl-(S)-Cbz-BMI: (S)-1-(Benzoylcarbonyl)-2-tert.-butyl-3,5-dimethyl-4-imidazolidinon oder Methyl-(S)-BDI: (S)-tert.-Butyl-2-tert.-butyl-5-methyl-4-methoxy-2-hydroimidazol-1-carboxylat umgesetzt.
Die Umsetzung kann in Anwesenheit von Lithiumdiisopropylamin erfolgen.

Als Lösungsmittel kann Tetrahydrofuran oder ein Ether, vorzugsweise Diethylether oder mindestens eine Komponente davon eingesetzt werden.

Die resultierende Verbindung g wird in Schritt vii an der Funktion OR² entschützt.
Hierzu kann beispielsweise Pyridinium-p-toluolsulfonsäure verwendet werden. Jedoch kann jedes bekannte Verfahren zur Entfernung der Schutzgruppe eingesetzt werden, wie beispielsweise der Einsatz von Säuren oder MgBr₂.

Als Lösungsmittel kommen Alkohole, wie Ethanol, Methanol, Propanol oder Isopropanol in Betracht.

Das Reaktionsprodukt h wird in Schritt viii zu einem Aldehyd oxidiert.

Hierzu können bekannte, milde Oxidationsverfahren eingesetzt werden.

Hierzu kann beispielhaft eine Oxidation nach Swern durchgerührt werden. Die Umsetzung erfolgt mit Oxalylchlorid, Dimethylsulfoxid in Anwesenheit von Triethylamin.

Die Reaktion findet in einem Bereich von -20°C bis -80°C, -30°C bis -80°C oder bevorzugt, zwischen -50°C bis -80°C statt. Typischerweise bei Trockeneistemperatur von ca. -78°C.

Als Lösungsmittel kann ein halogenierter Kohlenwasserstoff, wie Dichlormethan verwendet werden.

Reaktionsprodukt ist der erfindungsgemäße Vorläufer nach Formel 3.

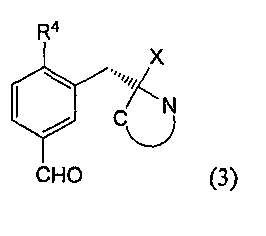

In einer weiteren Umsetzung kann der Vorläufer nach Formel 3 zu 2-[¹⁸F]FPhe oder 6-[¹⁸F]FMT umgesetzt werden.

Hierzu wird die Position des Substituenten R⁴ des Vorläufers gemäß Formel 3 ¹⁸F-fluoridiert. Diese Fluoridierung kann durch Standardverfahren erfolgen. Dabei können die Phasentransferkatalysatoren Kryptofix-Kaliumoxalat, Kryptofix-Kaliumcarbonat oder Tetrabutylammoniumhydrogencarbonat als Anionenaktivator für ¹⁸F- eingesetzt werden.

Das ¹⁸-fluorierte Zwischenprodukt wird in einem weiteren Schritt abgetrennt. Das ¹⁸-fluorierte Zwischenprodukt wird im Falle des 6-[¹⁸F]FMT zu einem Ester oxidiert.

Die Abtrennung kann durch Festphasenextraktion erfolgen. Hierzu wird das Reaktionsgemisch über eine Reverse-phase-Kartusche gereinigt.

Die Oxidation der Aldehydgruppe kann beispielsweise mit mCPBA, oder Peressigsäure oder Perborat durchgeführt werden, jedoch kommen auch andere Oxidationsmittel in Betracht. Als Lösungsmittel können halogenierte Kohlenwasserstoffe, wie Chloroform oder Methylenchlorid eingesetzt werden.

Im Falle des 2-[¹⁸F]FPhe wird an Stelle der Oxidation eine Decarbonylierung durchgeführt.

Geeignete Katalysatoren für die Decarbonylierung enthalten zweckmäßig vorzugsweise ein oder mehrere Übergangsmetalle der I., II., VI., VII. und VIII. Nebengruppe, wie Chrom, Mangan, Nickel, Kupfer oder Zink, bevorzugt jedoch ein oder mehrere Metalle aus der Gruppe der Platin-Metalle, insbesondere Rhodium. Dabei kann im heterogenen System mit festen, auf Trägern befindlichen Katalysatoren oder im homogenen System in flüssiger Phase gearbeitet werden.

Lösliche Rhodium-Komplexe, mit denen in einem homogenen flüssigen System gearbeitet werden kann oder mit denen Träger imprägniert werden können, sind beispielsweise Rhodium(I)-Komplexe, wie CIRh(PPh₃)₃ ("Wilkinson- Katalysator"), CIRh(CO) (PPh₃)₂, [CIRh(CO)₂]₂, acacRh(CO) (PPh₃), acacRh(CO)₂, (C₅H₅)Rh(C₈H₁₄) und (C₃H₅)Rh(PPh₃), wobei Ph für Phenyl, acac für Acetylacetonat, C₈H₁₄ für Cycloocten, C₅H₅ für Cyclopentadienyl und C₃H₅ für Allyl stehen. Geeignet sind auch Rhodium(II)- und Rhodium(III)-Komplexe, wie Rhodium(II)-acetat, Rhodium(II)-2,4-difluor-benzoat, Rh(acac)₃, RhCl₃'xH₂O, Rh(NO₃)₃ und (C₃H₅)PhCl₂(PPh₃)₂, Zu diesen Rhodium-Komplexen können vorteilhaft noch Verbindungen, die als Liganden wirken können, wie Phosphane, Phosphite oder Amine, zugesetzt werden.

In einem weiteren Schritt wird das erhaltene Produkt der Oxidation oder Decarbonylierung einer Hydrolyse unterzogen, wobei 2-[¹⁸F]FPhe oder 6-[¹⁸F]FMT oder deren α-methyliertes Derivat erhalten wird.

Die Hydrolyse kann in wässriger, vorzugsweise konzentrierter HI oder HBr oder in einer Lösung mit KI und HBr durchgeführt werden.

Eine Abtrennung des Produktes ist durch HPLC möglich.

Gegenstand der Erfindung ist auch die Verbindung nach Formel 3 bei der
X = H oder CH₃ und
R⁴= nukleophile Abgangsgruppe, z. B. F, Br, Cl, NO₂, -NR₃⁺ mit R = Alkyl, z. B. CH₃, C₂H₅, und
R³= (S)-BOC-BMI: (S)-1-(tert.-Butoxycarbonyl)-2-tert.-butyl-3-methyl-4- imidazolidinon , (S)-Cbz-BMI: (S)-1-Benzoylcarbonyl)-2-tert.-butyl-3-methyl-4-midazolidinon, (S)-BDI: (S)-tert.-Butyl 2-tert.-utyl-4-methoxy-2,5-dihydroimidazol-1-carboxylat, Methyl-(S)-BOC-BMI: (2S,SR)-tert.-butyl-2-tert.-butyl-3,5-dimethyl-4-oxoimidazolidin-1-carboxylat ,Methyl-(S)-Cbz-BMI: (S)-1-(Benzoylcarbonyl)-2-tert.-butyl-3,5-dimethyl-4-imidazolidinon oder Methyl-(S)-BDI: (S)-tert.-Butyl-2-tert.-butyl-5-methyl-4-methoxy-2-hydroimidazol-1-carboxylat ist.

Formelschreibweise:

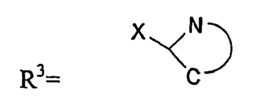

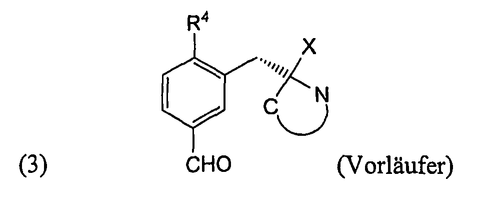

Durch Einsatz des neuen Markierungsvorläufers gelingt die Darstellung eines enantiomerenreinen Produktes nach Formel 1 und 2 (ee ≥ 98%) über eine nukleophile Synthese mit nur drei radioaktiven Schritten. Damit wird eine automatisierte Routinesynthese für die Verbindungen nach Formel 1 und 2 praktikabel. Der Vorläufer wird ebenfalls mit einer Enantiomerenreinheit von ≥ 98% erhalten.

Das erfindungsgemäße Verfahren und der Vorläufer nach Formel 3 ermöglicht eine Synthese, die ab der Nuklidproduktion von ¹⁸F- fern bedient oder vollständig automatisiert zu betreiben ist.

Apparaturen für den automatisierten Synthesebetrieb umfassen in der Regel ein Vorlagegefäß, in welches aus Vorratsgefäßen, welche mit dem Vorlagegefäß über Zuleitungen in Verbindung stehen über eine Steuerung mit Reagentien beschickt wird. Die Befüllung und Entleerung des Vorlagegefäßes erfolgt in der Regel durch Erzeugung eines Überdrucks oder eines Unterdrucks. Beispielhaft kann die kommerziell verfügbare Vorrichtung TRACERlab FX F-N genannt werden, welche neben Vorratsgefäßen für Reagentien, einem Vorlagegefäß aus Glaskohlefaser und Magnetrührer sowie herausziehbarer Nadel, mit einem Aktivitätsdetektor sowie einem Vakuumsystem mit Kühlfalle ausgestattet ist. Die Vorrichtung besitzt eine ¹⁸O-Wasser-Aufarbeitung sowie eine Festphasenextraktionseinheit mit präparativer HPLC, zwei HPLC-Eluenten und HPLC Flusskontrolle, UV und Radioaktivitätsdetektoren für die HPLC, Auffangbehälter für Fraktionen, Festphasenextraktion sowie eine HPLC-Lösungsmittelrückführung. Ähnliche Vorrichtungen sind aus "One-step high-radiochemicalyield synthesis of [18F]FP-CIT using a protic solvent system" in Nuc. Med. Biol., 2007; 34: 345-351 von S. Lee, S Oh, D.Chi, S. Kang, H. Kil, J Kim, D. Moon und weiterhin von Chen X, Park r, Shahinian AH, et al. -labeled RGD peptide: initial evaluation for imaging brain tumor angiogenesis /Nuc. Med. Biol.. 2004; 31: 179-189 bekannt.

Der erfindungsgemäße Vorläufer ermöglicht eine vollautomatisierte Umsetzung einer solchen Anlage, in dem eine ¹⁸F-Fluoridierung des Vorläufers nach Formel 3 durchgeführt wird und danach die Aufarbeitung zum Endprodukt, nämlich zu 2-[¹⁸F]FPhe oder 6-[¹⁸F]FMT, erfolgt. 2-[¹⁸F]FPhe und 6-[¹⁸F]FMT werden in einer Enantiomere-NReinheit von ≥ 98% erhalten.

### Beispiel I:

Die Synthese gemäß der Figur 1 kann mit folgenden Reagenzien durchgeführt werden:
i) Methanol, Natriumborhydrid
ii) Dichlormethan, Dihydropyan, p-Toluolsulfonsäure
iii) Tetrahydropyran, sec.-Butyllithium, Dimethylformamid
iv) Methanol, Natriumborhydrid
v) Dichlormethan, Tetrabrommethan, Triphenylphosphin
vi) Tetrahydropyran, Lithiumdiisopropylamin, (S)-Boc-BMI
vii) Ethanol, Pyridiunium-p-toluolsulfonsäure
viii) Dichlormethan, Oxalylchlorid, Dimethylsulfoxid, Triethylamin

### Spezielles Ausführunsgbeispiel:

### (3-Brom-4-fluor-phenyl)-methanol

Eine Lösung von 5g (24,6 mmol) 3-Brom-4-fluor-benzaldehyd in 20 ml wasserfreiem Methanol wird unter Rühren portionsweise mit 1,38g (36,58 mmol) Natriumborhydrid versetzt und 1 Stunde bei RT gerührt. Nach Zugabe von Wasser wird mit Diethylether extrahiert und die organische Phase über Natriumsulfat getrocknet. Nach Entfernung des Lösungsmittels im Vakuum erhält man den entsprechenden Alkohol. Das Produkt wird in reiner Form erhalten und kann für weitere Reaktionen eingesetzt werden.

| | |
|---|---|
| Form: | farblose Kristalle |
| Ausbeute: | 4,68 g (22,9 mmol; 93 %) |
| R_{f}: | 0,41 (Diethylether/n-Hexan = 1:1) |

### 2-(3-Brom-4-fluor-benzyloxy)-tetrahydropyran

Eine Lösung von 5 g (24,39 mmol) (3-Brom-4-fluor-phenyl)-methanol und 5,6 ml (60,98 mmol) 3,4 Dihydro-2H-pyran in 50 ml absolutem Dichlormethan wird bei 0°C mit einer Spatelspitze Toluolsulfonsäure Monohydrat versetzt und 15 min gerührt. Nach 2 stündigem Rühren bei RT wird mit Diethylether versetzt. Die organische Phase wird abgetrennt, mit Natriumchlorid- und Natriumcarbonat-Lösung sowie Wasser und nochmals mit Natriumchlorid-Lösung gewaschen, über Natriumsulfat getrocknet und das Lösungsmittel im Vakuum entfernt. Das erhaltene Rohprodukt wird säulenchromatographisch an Kieselgel unter Verwendung von Diethylether/n-Hexan (1/3) gereinigt.

| | |
|---|---|
| Form: | farbloses Öl |
| Ausbeute: | 6,42 g (22,19 mmol; 91 %) |
| R_{f}: | 0,78 (Diethylether/n-Hexan = 1:1) |

### 2-Fluor-5-(tetrahydropyran-2-yloxymethyl)-benzaldehyd

6,42 g (22,19 mmol) 2-(3-Brom-4-fluor-benzyloxy)-tetrahydropyran werden in 50 ml absolutem THF gelöst, bei -78°C unter Argonatmosphäre langsam mit 19,2 ml sec.-BuLi (1,4 M in Cyclohexan) versetzt und 45 min gerührt. Nach Zugabe von 2,6 ml (33,83 mmol) DMF rührt man die Reaktionslösung weitere 60 min bei RT. Nach Zugabe von Wasser wird mit Diethylether extrahiert und die organische Phase über Natriumsulfat getrocknet. Nach Entfernung des Lösungsmittels im Vakuum wird das Rohprodukt anschließend säulenchromatographisch an Kieselgel unter Verwendung von Diethylether/n-Hexan (1/1) gereinigt.

| | |
|---|---|
| Form: | gelbes Öl |
| Ausbeute: | 2,5 g (10,49 mmol; 47 %) |
| R_{f}: | 0,68 (Diethylether/n-Hexan= 1:1) |

### [2-Fluor-5-(tetrahydropyran-2-yloxymethyl)-phenyl]-methanol

Eine Lösung von 2,5 g (10,49 mmol) 2-Fluor-5-(tetrahydropyran-2-yloxymethyl)-benzaldehyd in wasserfreiem Methanol wird unter Rühren portionsweise mit 0,60 g (15,7 mmol) Natriumborhydrid versetzt und 1 Stunde bei RT gerührt. Nach Zugabe von Wasser wird mit Diethylether extrahiert und die organische Phase über Natriumsulfat getrocknet. Nach Entfernung des Lösungsmittels im Vakuum wird der Benzylalkohol in reiner Form erhalten.

| | |
|---|---|
| Form: | farbloses Öl |
| Ausbeute: | 2,04 g (8,49 mmol; 81 %) |
| R_{f}: | 0,25 (Diethylether/n-Hexan = 1:1) |

### 2-(3-Brommethyl-4-fluor-benzyloxy)-tetrahydropyran

Zu einer eiskalten Lösung von 3 (12,49 mmol) [2-Fluor-5-(tetrahydropyran-2-yloxymethyl)-phenyl]methanol und (15,61 mmol) Tetrabormmethan in 30 ml wasserfreiem Dichlormethan werden 4,44 g (16,94 mmol) Triphenylphosphin in 10 ml Dichlormethan zugetropft und weitere 45 min bei 0°C gerührt. Die Reaktionslösung wird mit Pentan versetzt, der Niederschlag abgesaugt und mit Dichlormethan gewaschen. Das Filtrat wird mit 5% Natriumhydrogencarbonatlösung, Wasser und Natriumchloridlösung gewaschen und über Magnesiumsulfat getrocknet. Nach der Entfernung des Lösungsmittels wird der Rückstand an Kieselgel unter Verwendung von Diethylether/Petrolether 1:10 säulenchromatographisch gereinigt.

| | |
|---|---|
| Form: | farbloses Öl |
| Ausbeute: | 1,85 g (6,1 mmol; 49 %) |
| R_{f}: | 0,21 (Diethylether/Petrolether = 1:10) |

### (25,5S)-2-tert.-Butyl-5-[2-fluor-5-(tetrahydro-2H-pyran-2-yloxymethyl)-benzyl]-3-methyl-4-oxo-imidazolin-1-carbonsäure-tert.-butyl ester

Zu einer Lösung von 1 g (3,9 mmol) (S)-BOC-BMI in 20 ml wasserfreiem THF werden bei - 78 °C unter Argonatmosphäre 2,6 ml (3,9 mmol) LDA gegeben und 40 min gerührt. Nach Zugabe von 1,18 g (3,9 mmol) 2-(3-Brommethyl-4-fluor-benzyloxy)-tetrahydropyran wird die Reaktionslösung 18 Stunden bei RT gerührt, mit gesättigter Ammoniumchlorid-Lösung versetzt und in Diethylether und Wasser aufgenommen. Die wässrige Phase wird zweimal mit Diethylether extrahiert, die vereinigten organischen Extrakte über Natriumsulfat getrocknet und das Lösungsmittel im Vakuum reduziert. Das Rohprodukt wird säulenchromatographisch an Kieselgel unter Verwendung von Diethylether/Petrolether 2:1 gereinigt.

| | |
|---|---|
| Form: | farbloses Öl |
| Ausbeute: | 0,43 g (0,89 mmol; 23%) |
| R_{f}: | 0,58 (Diethylether/Petrolether = 2:1) |

### (25,5S)-2-tert.-Butyl-5-(2-fluor-5-hydroxymethyl-benzyl)-3-methyl-4-oxo-imidazolin-1-carbonsäure-tert.-butyl ester

0,43 g (0,89 mmol) (2S,5S)-2-tert.-Butyl-5-[2-fluor-5-(tetrahydro-2H-pyran-2-yloxymethyl)-benzyl]-3-methyl-4-oxo-imidazolin-1-carbonsäure-tert.-butyl ester werden in 10 ml Ethanol gelöst und mit 23 mg (0,09 mmol) Pyridinium-p-toluolsulfonat über Nacht bei 60 °C gerührt. Nach Abkühlung wird das Reaktionsgemisch in Diethylether aufgenommen, mit Natriumchloridlösung gewaschen und über Natriumsulfat getrocknet. Die flüchtigen Bestandteile werden anschließend im Vakuum entfernt.

| | |
|---|---|
| Ausbeute: | 0,34 g (0,88 mmol; 99 %) |
| Form: | farbloses Öl |
| R_{f}: | 0,29 (Diethylether/n-Hexan = 10:1) |

### (25,5S)-2-tert.-Butyl-5-(2-fluor-5-formyl-benzyl)-3-methyl-4-oxo-imidazolin-1-carbonsäure-tert.-butyl ester

Unter Argonatmosphäre werden 22 µl (0,26 mmol) Oxalylchlorid in 2 ml Dichlormethan bei -60°C langsam mit 41 µl (0,58 mmol) Dimethylsulfoxid versetzt und 10 min gerührt. Nach Zugabe von 94 mg (0,24 mmol) 2-tert.-Butyl-5-(2-fluor-5-hydroxymethyl-benzyl)-3-methyl-4-oxo-imidazo-lin-1-carbonsäure-tert.-butyl ester in 5 ml Dichlormethan wird weitere 15 min gerührt, die Reaktionslösung mit 167µl (1,2 mmol) Triethylamin versetzt, langsam auf RT erwärmt und nach Zugabe von 5 ml Wasser für weitere 10 min gerührt. Die wässrige Phase wird abgetrennt und mit Dichlormethan extrahiert. Die organische Phase wird über Natriumsulfat getrocknet und das Lösungsmittel im Vakuum entfernt. Das Rohprodukt wird an Kieselgel mit Diethylether/n-Hexan 5:1 chromatographiert.

| | |
|---|---|
| Form: | farbloser Feststoff |
| Ausbeute: | 92 mg (0,23 mmol; 98%) |
| R_{f}: | 0,51 (Diethylether/n-Hexan = 5:1) |

## Patentansprüche

1. Verbindung der allgemeinen Formel (3) 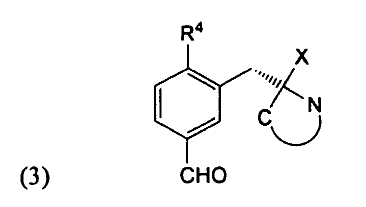 mit X = H oder CH₃ und
R⁴= nukleophile Abgangsgruppe, z. B. F, Br, Cl, NO₂, -NR₃⁺ mit R = Alkyl, z. B. CH₃, C₂H₅ und 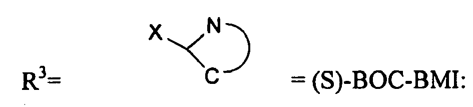 (S)-1-(tert.-Butoxycarbonyl)-2-tert.-butyl-3-methyl-4- imi-dazolidinon , (S)-Cbz-BMI: (S)-1-(Benzoylcarbonyl)-2-tert.-butyl-3-methyl-4-midazolidinon, (S)-BDI: (S)-tert.-Butyl 2-tert.-butyl-4-methoxy-2,5-dihydroimidazol-1-carboxylat, Methyl-(S)-BOC-BMI: (2S,5R)-tert.-Butyl-2-tert.-butyl-3,5-dimethyl-4-oxoimidazolidin-1-carboxylat, Methyl-(S)-Cbz-BMI: (S)-1-(Benzoylcarbonyl)-2-tert.-butyl-3,5-dimethyl-4-imidazolidinon oder Methyl-(S)-BDI: (S)-tert.-Butyl-2-tert.-butyl-5-methyl-4-methoxy-2-hydroimidazol-1-carboxylat.

2. Verfahren zur Herstellung der Verbindung nach Formel (3) 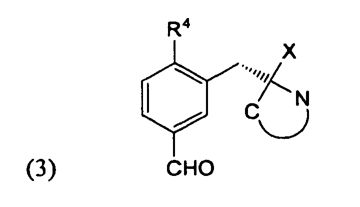 mit X = H oder CH₃ und
R⁴= nukleophile Abgangsgruppe, z. B. F, Br, Cl, NO₂, -NR₃⁺ mit R = Alkyl, z. B. CH₃, C₂H₅ und, 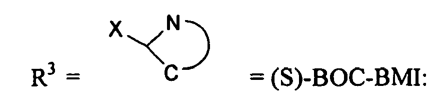 (S)-1-(tert.-Butoxycarbonyl)-2-tert.-butyl-3-methyl-4-imidazolidinon, (S)-Cbz-BMI: (S)-1-(Benzoylcarbonyl)-2-tert.-butyl-3-methyl-4-imidazolidinon, (S)-BDI: (S)-tert.-Butyl 2-tert.-butyl-4-methoxy-2,5-dihydroimidazol-1-carboxylat, Methyl-(S)-BOC-BMI: (2S,5R)-tert.-Butyl-2-tert.-butyl-3,5-dimethyl-4-oxoimidazolidin-1-carboxylat, Methyl-(S)-Cbz-BMI: (S)-1-(Benzoylcarbonyl)-2-tert.-butyl-3,5-dimethyl-4-imidazolidinon oder Methyl-(S)-BDI oder (S)-tert.-Butyl-2-tert.-butyl-5-methyl-4-methoxy-2-hydroimidazol-1-carboxylat ist, bei dem eine Verbindung (b) in einem Schritt (ii) 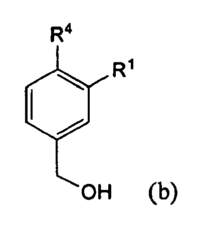 mit R⁴ = nukleophile Abgangsgruppe, z. B. F, Br, Cl, NO₂, -NR₃⁺ mit R = Alkyl; z. B. CH₃, C₂H₅,
R¹ = Br oder I ist
zu Verbindung (c) durch Einführen einer Schutzgruppe 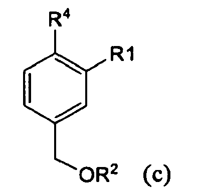 mit R² = Tetrahydropyranyl (THP), Methylthiomethyl (MTM), Methoxymethyl (MOM) TBDMS, TBDPS, allgemein Silylschutzgruppen umgesetzt wird;
wonach bei Verbindung (c) in einem weiteren Schritt (iii) der Substituent R¹ durch eine Formylgruppe ersetzt zu Verbindung (d) 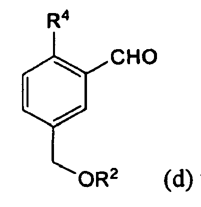 wird,
in Verbindung (d) die Formylgruppe in einem Schritt (iv) zu einem Alkohol reduziert wird, wobei das Produkt (e) 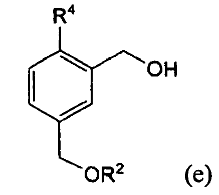 entsteht,
Verbindung nach Formel (e) in einem weiteren Schritt (v) zu der Verbindung (f) halogeniert wird , wobei alternativ statt der Br Gruppe eine Tosylgruppe durch Tosylierung eingeführt werden kann , 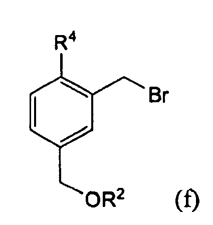 der in einer weiteren Reaktion (vi)
zu dem Produkt (g) 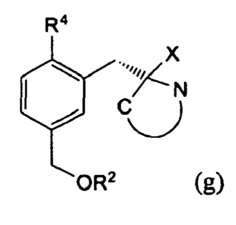 mit einem chrialen Reagenz R³ aus der Gruppe (S)-BOC-BMI: (S)-1-(tert.-Butoxycarbonyl)-2-tert.-butyl-3-methyl-4-imidazolidinon, (S)-Cbz-BMI: (S)-1-(Benzoylcarbonyl)-2-tert.-butyl-3-methyl-4-imidazolidinon, (S)-BDI: (S)-tert.-Butyl 2-tert.-butyl-4-methoxy-2,5-dihydroimidazol-1-carboxylat, Methyl-(S)-BOC-BMI: (25,5R)-tert.-Butyl-2-tert.-butyl-3,5-dimethyl-4-oxoimidazolidin-1-carboxylat, Methyl-(S)-Cbz-BMI: (S)-1-(Benzoylcarbonyl)-2-tert.-butyl-3,5-dimethyl-4-imidazolidinon oder Methyl-(S)-BDI oder (S)-tert.-Butyl-2-tert.-butyl-5-methyl-4-methoxy-2-hydroimidazol-1-carboxylat umgesetzt wird,
die Verbindung (g) in einem weiteren Schritt (vii) durch Abspalten der Schutzgruppe, R² zu Verbindung (h) 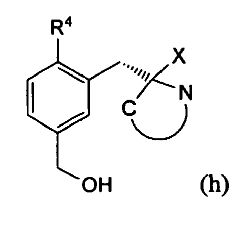 mit 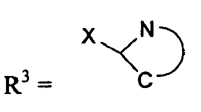 als Formelschreibweise
umgesetzt wird, die in Schritt (viii) an der Alkoholfunktion durch Oxidation zu dem Aldehyl nach Formel (3) 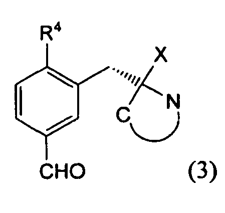 umgesetzt wird.

## Claims

1. Compound of the general formula (3) 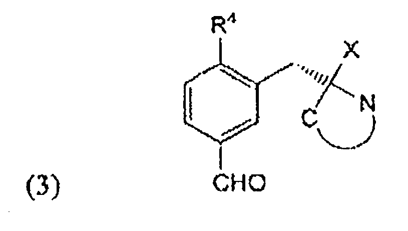 with X = H or CH₃ and
R⁴ = a nucleophilic leaving group, such as F, Br, Cl, NO₂ or -NR₃⁺ for example, with R = alkyl, such as CH₃ or C₂H₅ for example, and 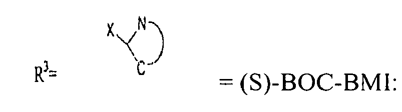 (S)-1-(tert.-butoxycarbonyl)-2-tert.-butyl-3-methyl-4-imidazolidinone, (S)-Cbz-BMI: (S)-1-(benzoyl carbonyl)-2-tert.-butyl-3-methyl-4-imidazolidinone, (S)-BDI: (S)-tert.-butyl 2-tert.-butyl-4-methoxy-2,5-dihydroimidazole-1-carboxylate, methyl-(S)-BOC-BMI: (2S, 5R)-tert.-butyl-2-tert.-butyl-3,5-dimethyl-4-oxoimidazolidine-1-carboxylate, methyl-(S)-Cbz-BMI: (S)-1-(benzoyl carbonyl)-2-tert.-butyl-3,5-dimethyl-4-imidazolidinone or methyl-(S)-BDI: (S)-tert.-butyl-2-tert.-butyl-5-methyl-4-methoxy-2-hydroimidazole-1-carboxylate.

2. Method for producing the compound according to the formula (3) 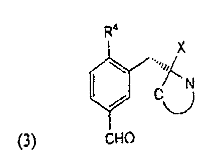 with X = H or CH₃ and
R⁴ = a nucleophilic leaving group, such as F, Br, Cl, NO₂ or -NR₃⁺ for example, with R = alkyl, such as CH₃ or C₂H₅ for example, and 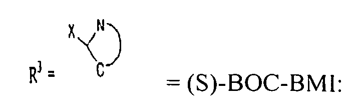 (S)-1-(tert.-butoxycarbonyl)-2-tert.-butyl-3-methyl-4-imidazolidinone, (S)-Cbz-BMI: (S)-1-(benzoyl carbonyl)-2-tert.-butyl-3-methyl-4-imidazolidinone, (S)-BDI: (S)-tert.-butyl 2-tert.-butyl-4-methoxy-2,5 dihydroimidazole-1-carboxylate, methyl-(S)-BOC-BMI: (2S, 5R)-tert.-butyl-2-tert.-butyl-3,5-dimethyl-4-oxoimidazolidine-1-carboxylate, methyl-(S)-Cbz-BMI: (S)-1-(benzoyl carbonyl)-2-tert.-butyl-3,5-dimethyl-4-imidazolidinone or methyl-(S)-BDI or (S)-tert.-butyl-2-tert.-butyl-5-methyl-4-methoxy-2-hydroimidazole-1-carboxylate,
in which a compound (b) is converted into compound (c) in a step (ii) 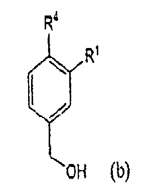 with R⁴ = a nucleophilic leaving group, such as F, Br, Cl, NO₂ or -NR₃⁺ for example, with R = alkyl, such as CH₃ or C₂H₅ for example,
R¹= Br or I
by introducing a protecting group 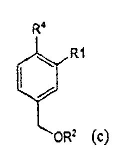 with R² = tetrahydropyranyl (THP), methyl thiomethyl (MTM) or methoxymethyl (MOM), TBDMS, TBDPS or silyl protecting groups in general;
according to which in compound (c) the substituent R¹ is substituted by a formyl group to compound (d) in a further step (iii), 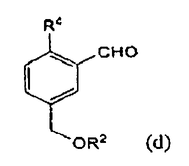 in compound (d) the formyl group is reduced to an alcohol in a step (iv), in which the product (e) occurs, 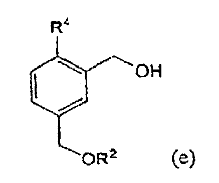 the compound according to formula (e) is halogenated to compound (f) in a further step (v), in which alternatively instead of the Br group a tosyl group may be introduced by tosylation, 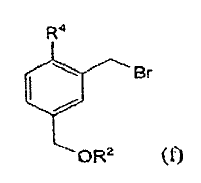 which is converted into product (g) in a further reaction (vi) 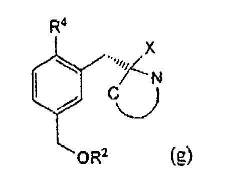 with a chiral reagent R³ from the group (S)-BOC-BMI: (S)-1-tert.-butoxycarbonyl)-2-tert.-butyl-3-methyl-4-imidazolidinone, (S)-Cbz-BMI: (S)-1-(benzoyl carbonyl)-2-tert.-butyl-3-methyl-4-imidazolidinone, (S)-BDI: (S)-tert.-butyl 2-tert.-butyl-4-methoxy-2,5-dihydroimidazole-1-carboxylate, methyl-(S)-BOC-BMI: (2S, 5R)-tert.-butyl-2-tert.-butyl-3,5-dimethyl-4-oxoimidazolidine-1-carboxylate, methyl-(S)-Cbz-BMI: (S)-1-(benzoyl carbonyl)-2-tert.-butyl-3,5-dimethyl-4-imidazolidinone or methyl-(S)-BDI or (S)-tert.-butyl-2-tert.-butyl-5-methyl-4-methoxy-2-hydroimidazole-1-carboxylate, the compound (g) is converted into compound (h) in a further step (vii) by separating off the protecting group R² 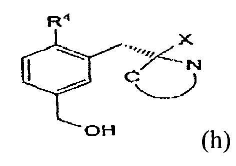 with 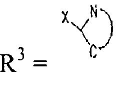 as the way of writing the formula,
which is converted into the aldehyle according to formula (3) in step (viii) by oxidation on the alcohol function,

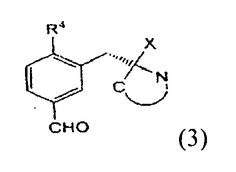

## Revendications

1. Composé de formule générale (3) 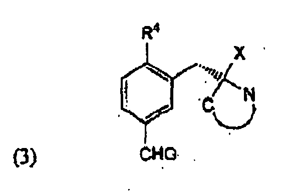 dans laquelle X = H ou CH₃ et
R⁴ = groupes sortants nucléophiles, par exemple F, Br, Cl, NO₂, -NR₃⁺ où R = alkyle, par exemple CH₃, C₂H₅ et 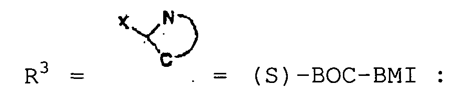 (S)-1-(tert.-butoxycarbonyl)-2-tert.-butyl-3-méthyl-4-imidazolidinone, (S)-Cbz-BMI : (S)-1-(benzoylcarbonyl)-2-tert.-butyl-3-méthyl-4-imidazolidinone, (S)-BDI : (S)-tert.-butyl-2-tert.-butyl-4-méthoxy-2,5-dihydroimidazol-1-carboxylate, méthyl-(S)-BOC-BMI : (2S,5R)-tert.-butyl-2-tert.-butyl-3,5-diméthyl-4-oxo-imidazolidine-1-carboxylate, méthyl-(S)-Cbz-BMI : (S)-1-(benzoylcarbonyl)-2-tert.-butyl-3,5-diméthyl-4-imidazolidinone ou méthyl-(S)-BDI : (S)-tert.-butyl-2-tert.-butyl-5-méthyl-4-méthoxy-2-hydroimidazol-1-carboxylate.

2. Procédé de préparation du composé de formule générale (3) 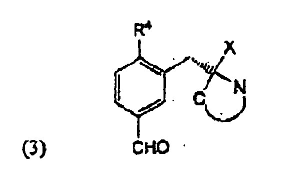 dans laquelle X = H ou CH₃ et
R⁴ = groupes sortants nucléophiles, par exemple F, Br, Cl, NO₂, -NR₃⁺ où R = alkyle, par exemple CH₃, C₂H₅ et 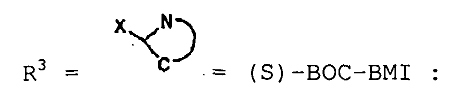 (S)-1-(tert.-butoxycarbonyl)-2-tert.-butyl-3-méthyl-4-imidazolidinone, (S)-Cbz-BMI : (S)-1-(benzoylcarbonyl)-2-tert.-butyl-3-méthyl-4-imidazolidinone, (S)-BDI : (S)-tert.-butyl-2-tert.-butyl-4-méthoxy-2,5-dihydroimidazol-1-carboxylate, méthyl-(S)-BOC-BMI : (2S,5R)-tert.-butyl-2-tert.-butyl-3,5-diméthyl-4-oxo-imidazolidine-1-carboxylate, méthyl-(S)-Cbz-BMI : (S)-1-(benzoylcarbonyl)-2-tert.-butyl-3,5-diméthyl-4-imidazolidinone ou méthyl-(S)-BDI : (S)-tert.-butyl-2-tert.-butyl-5-méthyl-4-méthoxy-2-hydroimidazol-1-carboxylate,
dans lequel, au cours d'une étape (ii), on fait réagir un composé (b) 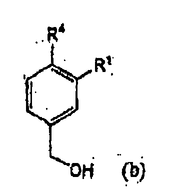 dans lequel R⁴ = groupes sortants nucléophiles, par exemple F, Br, Cl, NO₂, -NR₃⁺ où R = alkyle, par exemple CH₃, C₂H₅,
R¹ = Br ou I
pour donner un composé (c) par introduction d'un groupe protecteur 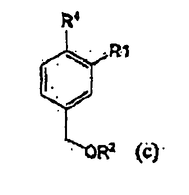 dans lequel R² = tétrahydropyranyle (THP), méthylthiométhyle (MTM), méthoxyméthyle (MOM), TBDMS, TBDPS, généralement des groupes protecteurs silyles ;
après quoi, dans le composé (c), le substituant R¹ est remplacé par un groupe formyle, au cours d'une étape supplémentaire (iii), pour donner le composé (d) 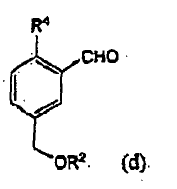 le groupe formyle dans le composé (d) est réduit, au cours d'une étape (iv), en un alcool, donnant le produit (e) 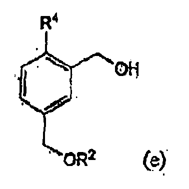 le composé selon la formule (e) est halogéné au cours d'une étape supplémentaire (v) pour donner le composé (f), où, en variante, un groupe tosyle peut être introduit par tosylation à la place du groupe Br, 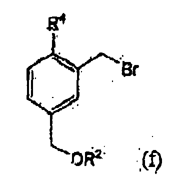 que l'on fait réagir dans une réaction ultérieure (vi) pour donner le produit (g) 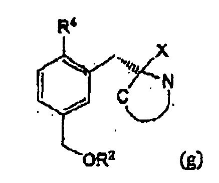 avec un réactif chiral R³ choisi dans le groupe (S)-BOC-BMI : (S)-1-(tert.-butoxycarbonyl)-2-tert.-butyl-3-méthyl-4-imidazolidinone, (S)-Cbz-BMI : (S)-1-(benzoylcarbonyl)-2-tert.-butyl-3-méthyl-4-imidazolidinone, (S)-BDI : (S)-tert.-butyl-2-tert.-butyl-4-méthoxy-2,5-dihydroimidazol-1-carboxylate, méthyl-(S)-BOC-BMI : (2S,5R)-tert.-butyl-2-tert.-butyl-3,5-diméthyl-4-oxo-imidazolidine-1-carboxylate, méthyl-(S)-Cbz-BMI : (S)-1-(benzoylcarbonyl)-2-tert.-butyl-3,5-diméthyl-4-imidazolidinone ou méthyl-(S)-BDI : (S)-tert.-butyl-2-tert.-butyl-5-méthyl-4-méthoxy-2-hydroimidazol-1-carboxylate,
on fait réagir le composé (g), au cours d'une autre étape (vii), par clivage du groupe protecteur R² pour donner le composé (h) 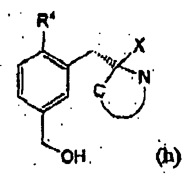 dans lequel 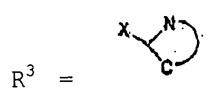 en notation sous forme de formule, que l'on fait réagir, au cours de l'étape (viii), par oxydation sur la fonction alcool pour donner l'aldéhyde selon la formule (3)
